# EUROPEAN PATENT APPLICATION

(11) **EP 4 230 724 A1**
(43) Date of publication of application: **23.08.2023**
(21) Application number: 21880196.7
(22) Date of filing: 14.10.2021
(51) Int. Cl.: C12N 5/07, C12N 5/077

(54) **CULTURE MEDIUM FOR PRODUCING EXTRACELLULAR VESICLES, CULTURE MEDIUM KIT, ADDITIVE, AND METHOD FOR PRODUCING EXTRACELLULAR VESICLES**

(30) Priority: 16.10.2020 JP 2020174347; 26.03.2021 JP 2021053337
(71) Applicant: FUJIFILM Corporation, Tokyo 106-8620 (JP)
(72) Inventor: YAMANE Masayuki, Amagasaki-shi, Hyogo 661-0963 (JP); ARAI Hanako, Amagasaki-shi, Hyogo 661-0963 (JP)
(74) Representative: Hoffmann Eitle
(86) International application number: PCT/JP2021/038095
(87) International publication number: WO 2022/080461

(57) **Abstract**

An object of the present invention is to provide a culture medium for producing extracellular vesicles, a culture medium kit, an additive, and a method for producing extracellular vesicles, whereby the production of extracellular vesicles is promoted. The present invention is to provide a culture medium for producing extracellular vesicles. The culture medium for producing extracellular vesicles contains a basal medium for culturing animal cells, and at least one component selected from L-glutamine or a salt thereof, transferrin, selenious acid or a salt thereof, insulin, insulin-like growth factors, a serotonin compound, and transforming growth factor β. Furthermore, in a case of containing L-glutamine or a salt thereof, the culture medium for producing extracellular vesicles contains L-glutamine or a salt thereof with a concentration of 5 mM or more in the culture medium for producing extracellular vesicles. The present invention is to further provide a culture medium kit, an additive, and a method for producing extracellular vesicles.

## Description

### BACKGROUND OF THE INVENTION

### 1. Field of the Invention

The present invention relates to a culture medium for producing extracellular vesicles, a culture medium kit, an additive, and a method for producing extracellular vesicles.

### 2. Description of the Related Art

It is considered that extracellular vesicles that are derived from cells and are small membrane vesicles composed of lipid bilayers are responsible for intercellular communication through the transfer of nucleic acids such as mRNA and microRNA contained, or proteins (Mathieu M, Martin-Jaular L, Lavieu G, Thery C (2019) Specificities of secretion and uptake of exosomes and other extracellular vesicles for cell-to-cell communication. Nat Cell Biol, 21(1): 9-17). In addition, for the purpose of diagnosing or treating diseases with extracellular vesicles, extracellular vesicles have been cultured and acquired.

As a method for acquiring extracellular vesicles produced (secreted) from mesenchymal stem cells, a method for acquiring extracellular vesicles from a cell culture supernatant liquid in which mesenchymal stem cells has been cultured, by an ultracentrifugation method or the like is known.

A culture medium (cell culture solution) to which fetal bovine serum (FBS) used for the purpose of supplying a nutritional component to cells is added is commonly used for culturing cells. However, it is not desirable to use a culture medium containing FBS for purification of extracellular vesicles because FBS contains abundant extracellular vesicles derived from fetal bovine.

Therefore, as the serum-free culture medium, D-MEM (containing 4 mM of L-glutamine) which is a basal medium is used in Basalova et al., (2020) Secretome of Mesenchymal Stromal Cells Prevents Myofibroblasts Differentiation by Transferring Fibrosis-Associated microRNAs within Extracellular Vesicles. Cells: 9 (5): 1272., and a culture medium in which 0.01 µg/mL of basic fibroblast growth factors (bFGFs) or the like is added to D-MEM (containing 4 mM of L-glutamine) which is a basal medium is used in Mesenchymal Stem Cell-Derived Extracellular Vesicles as Mediators of Anti-Inflammatory Effects: Endorsement of Macrophage Polarization.

### SUMMARY OF THE INVENTION

However, the present inventors produced extracellular vesicles by using D-MEM as a basal medium or a culture medium obtained by adding bFGF to D-MEM as a basal medium, and found that the extremely small amount of extracellular vesicles were produced, and the produced extracellular vesicles were insufficient for industrial use.

In view of the above circumstances, an object of the present invention is to provide a culture medium for producing extracellular vesicles, a kit, an additive, and a method for producing extracellular vesicles, whereby the production of extracellular vesicles is promoted.

As a result of diligent studies on components of the culture medium for producing extracellular vesicles in order to solve the above-described problems, the present inventors have found that using a culture medium for producing extracellular vesicles, which contains at least one component selected from L-glutamine or a salt thereof, transferrin, selenious acid or a salt thereof, insulin, insulin-like growth factors, a serotonin compound, and transforming growth factor β, and contains L-glutamine or a salt thereof with a concentration of 5 mM or more in the culture medium for producing extracellular vesicles in a case of containing L-glutamine or a salt thereof, enables the production of the extracellular vesicles to be promoted, and have completed the present invention.

That is, the present invention is a culture medium for producing extracellular vesicles, and the fundamental aspect thereof is follows.
(1) A culture medium for producing extracellular vesicles, comprising:
   a basal medium for culturing animal cells; and at least one component selected from L-glutamine or a salt thereof, transferrin, selenious acid or a salt thereof, insulin, insulin-like growth factors, a serotonin compound, and transforming growth factor β,
   in which a concentration of L-glutamine or a salt thereof is 5 mM or more in the culture medium for producing extracellular vesicles in a case of containing L-glutamine or a salt thereof.
(2) The culture medium for producing extracellular vesicles according to (1), in which the culture medium for producing extracellular vesicles contains the L-glutamine or a salt thereof.
(3) The culture medium for producing extracellular vesicles according to (1), in which the culture medium for producing extracellular vesicles contains the transferrin.
(4) The culture medium for producing extracellular vesicles according to (1), in which the culture medium for producing extracellular vesicles contains the selenious acid or a salt thereof.
(5) The culture medium for producing extracellular vesicles according to (1), in which the culture medium for producing extracellular vesicles contains the transferrin and the selenious acid or a salt thereof.
(6) The culture medium for producing extracellular vesicles according to (1), in which the culture medium for producing extracellular vesicles contains the insulin.
(7) The culture medium for producing extracellular vesicles according to (1), in which the culture medium for producing extracellular vesicles contains the insulin-like growth factors.
(8) The culture medium for producing extracellular vesicles according to (1), in which the culture medium for producing extracellular vesicles contains the serotonin compound.
(9) The culture medium for producing extracellular vesicles according to (1), in which the culture medium for producing extracellular vesicles contains the transforming growth factor β.
(10) The culture medium for producing extracellular vesicles according to any one selected from (1) to (9), in which the culture medium for producing extracellular vesicles does not contain a serum.
(11) The culture medium for producing extracellular vesicles according to any one selected from (1) to (10), in which the culture medium for producing extracellular vesicles contains at least two components selected from L-glutamine or a salt thereof, transferrin, selenious acid or a salt thereof, insulin, insulin-like growth factor, a serotonin compound, and transforming growth factor β.
(12) The culture medium for producing extracellular vesicles according to any one selected from (1) to (10), in which the culture medium for producing extracellular vesicles contains at least three components selected from L-glutamine or a salt thereof, transferrin, selenious acid or a salt thereof, insulin, insulin-like growth factors, a serotonin compound, and transforming growth factor β.

The present invention is also a culture medium kit for producing extracellular vesicles, and described in detail below.
(13) A culture medium kit for producing extracellular vesicles, comprising:
   a basal medium for culturing animal cells; and an additive for producing extracellular vesicles containing at least one component selected from L-glutamine or a salt thereof, transferrin, selenious acid or a salt thereof, insulin, insulin-like growth factors, a serotonin compound, and transforming growth factor β,
   in which in a case where the additive for producing extracellular vesicles contains L-glutamine or a salt thereof, the additive for producing extracellular vesicles contains L-glutamine or a salt thereof in an amount at which a concentration of the L-glutamine or a salt thereof in the culture medium for producing extracellular vesicles is 5 mM or more.

The present invention is also an additive for producing extracellular vesicles, and described in detail below.
(14) An additive for producing extracellular vesicles used to produce a culture medium,
   in which the culture medium is the culture medium according to any one selected from (1) to (12),
   the additive contains at least one component selected from L-glutamine or a salt thereof, transferrin, selenious acid or a salt thereof, insulin, insulin-like growth factors, a serotonin compound, and transforming growth factor β, and
   in a case where the additive for producing extracellular vesicles contains L-glutamine or a salt thereof, the additive for producing extracellular vesicles contains L-glutamine or a salt thereof in an amount at which a concentration of the L-glutamine or a salt thereof in the culture medium for producing extracellular vesicles is 5 mM or more.
   The present invention is also a method for producing extracellular vesicles, and described in detail below.
(15) A method for producing extracellular vesicles, the method comprising producing extracellular vesicles from mesenchymal stem cells by using the culture medium according to any one selected from (1) to (12).

According to the culture medium for producing extracellular vesicles, the kit, and the method for producing extracellular vesicles of the present invention, the production of extracellular vesicles is promoted.

### BRIEF DESCRIPTION OF THE DRAWINGS

Fig. 1 is a result of analyzing the number of particles of extracellular vesicles produced by using various media with a nanotracking analysis method.
Fig. 2 is a diagram illustrating primers used for quantitative PCR.
Fig. 3 is a diagram illustrating an evaluation result of the activity of the extracellular vesicles produced from mesenchymal stem cells by using various media with the mRNA expression level of Collagen III being used as an index.
Fig. 4 is a diagram illustrating an evaluation result of the activity of the extracellular vesicles produced from mesenchymal stem cells by using various media with the mRNA expression level of Collagen III being used as an index.

### DESCRIPTION OF THE PREFERRED EMBODIMENTS

In the present specification, in a case where the upper limit and the lower limit of a range are indicated, A to B means that equal to or greater than A and equal to or smaller than B, unless otherwise specified.

Extracellular vesicles according to an embodiment of the present invention refer to small membrane vesicles each of which is derived from a cell and composed of a lipid bilayer membrane. Each of the extracellular vesicles usually has a diameter of 20 nm to 1000 nm, preferably 50 nm to 800 nm, more preferably 50 nm to 500 nm, and particularly preferably 50 nm to 200 nm. Examples of the extracellular vesicles include extracellular vesicles variously classified according to the origin of their occurrence, the size of small membrane vesicles, and the like as described in Nature Reviews Immunology 9,581-593 (August 2009), "Obesity Research" Vol. 13, No. 2, 2007, Topics Naoto Aoki et al.. Specific examples thereof include exosomes, microvesicles, ectosomes, membrane particles, exosome-like vesicles, apoptotic vesicles, adiposomes, and the like, exosomes and microvesicles are preferable, and exosomes are more preferable.

The exosomes are cell-derived small membrane vesicles composed of lipid bilayers, and for example, each exosome has a diameter of 50 nm to 200 nm, preferably has a diameter of 50 nm to 150 nm, and more preferably has a diameter of 50 nm to 100 nm. The exosomes are believed to be derived from late endosomes. The microvesicles are cell-derived small membrane vesicles composed of lipid bilayers, and for example, each microvesicle has a diameter of 100 nm to 1000 nm, preferably has a diameter of 100 nm to 800 nm, and more preferably has a diameter of 100 nm to 500 nm. The microvesicles are believed to be derived from cell membranes.

Mesenchymal stem cells according to the present invention refer to stem cells having differentiation potential into cells belonging to (mesenchymal) tissues derived from mesoderm such as osteoblasts, adipocytes, muscle cells, and chondrocytes. The mesenchymal stem cells can be obtained by, for example, a method of separating mesenchymal stem cells from tissues derived from a mesoderm or a method of inducing mesenchymal stem cells from stem cells such as iPS cells and ES cells. As the mesenchymal stem cells according to the present invention, cells derived from one or more tissues selected from the group consisting of umbilical cord, umbilical cord blood, bone marrow, adipose, muscle, nerve, skin, endodontium, amniotic membrane, and placenta or cells derived from iPS cells are preferably used. Pretreatments such as recovery, concentration, purification, isolation, dilution with a buffer solution, and filtration sterilization may be performed on the mesenchymal stem cells. These pretreatments may be appropriately performed according to conventional methods. Examples of the origin of the mesenchymal stem cells according to the present invention include animals, and mammals are preferable, and humans are more preferable.

### <Culture Medium for Producing Extracellular Vesicles>

The culture medium for producing extracellular vesicles according to the embodiment of the present invention contains a basal medium for culturing animal cells and at least one component selected from L-glutamine, transferrin, selenic acids, insulin, insulin-like growth factors, serotonin, and transforming growth factor β (hereinafter, may be abbreviated as a component according to the present invention), and in a case of containing L-glutamine or a salt thereof, the culture medium for producing extracellular vesicles contains L-glutamine or a salt thereof with a concentration of 5 mM or more in the culture medium for producing extracellular vesicles. The culture medium for producing extracellular vesicles according to the embodiment of the present invention finally may contain a component contained in the basal medium for culturing animal cells according to the present invention, and a component according to the present invention (here, in a case of containing L-glutamine or a salt thereof, a concentration of L-glutamine or a salt thereof is 5 mM or more in the culture medium for producing extracellular vesicles) according to the present invention. For example, an additive that contains the component according to the present invention and contains L-glutamine or a salt thereof in an amount at which a concentration of the L-glutamine or a salt thereof in the culture medium for producing extracellular vesicles is 5 mM or more, in a case of containing L-glutamine or a salt thereof, the basal medium for culturing animal cells according to the present invention or the component of the basal medium for culturing animal cells according to the present invention, and water are mixed to obtain the culture medium for producing extracellular vesicles. The culture medium for producing extracellular vesicles according to the embodiment of the present invention is used to promote the production of extracellular vesicles. Specifically, as compared with, for example, a case of using D-MEM as a basal medium or a case of using a culture medium obtained by the addition of bFGF to D-MEM, the secretion of extracellular vesicles is promoted, and a larger number of extracellular vesicles can be obtained. The number of particles of the extracellular vesicles can be measured by, for example, a nanoparticle tracking analysis method (nano tracking analysis method). The culture medium for producing extracellular vesicles according to the embodiment of the present invention preferably contains no serum (serum-free culture medium) because the serum contains extracellular vesicles, and particularly preferably contains no FBS. Examples of the serum include fetal bovine serum and fetal calf serum (FBS and FCS), calf serum (CS), and horse serum (HS). The culture medium for producing extracellular vesicles according to the embodiment of the present invention may be an aqueous solution obtained by dissolution or dispersion of the component contained in the basal medium for culturing animal cells according to the present invention and the component according to the present invention (here, in a case of containing L-glutamine or a salt thereof, a concentration of L-glutamine or a salt thereof is 5 mM or more in the culture medium for producing extracellular vesicles) in a solvent such as water, which is in a liquid form (liquid medium) such as a dispersion liquid, or in a solid form (solid medium) such as agar (agar medium) or gel, and the liquid form (liquid medium) is preferable.

Examples of the L-glutamine or a salt thereof according to the present invention include L-glutamine and a halide of L-glutamine, and L-glutamine is preferable. A content of the L-glutamine or a salt thereof according to the present invention in the culture medium for producing extracellular vesicles according to the embodiment of the present invention is 5 mM or more, for example, 5 mM to 18 mM, preferably 5 mM to 16 mM, more preferably 5 mM to 14 mM, still more preferably 6 mM to 12 mM, and particularly preferably 6 mM to 10 mM. One or two or more kinds of the L-glutamine or a salt thereof according to the present invention may be used.

Examples of the transferrin according to the present invention include naturally occurring transferrin, recombinant transferrin (recombinant form), transferrin obtained by a chemical synthesis method, and the like. Examples of the organism from which the naturally occurring transferrin is derived include humans, mice, and the like. A content of the transferrin according to the present invention in the culture medium for producing extracellular vesicles according to the embodiment of the present invention is, for example, 0.01 mg/L to 1000 mg/L, preferably 0.05 mg/L to 500 mg/L, more preferably 0.1 mg/L to 100 mg/L, still more preferably 0.5 mg/L to 50 mg/L, and particularly preferably 1 mg/L to 10 mg/L.

As the selenious acid or a salt thereof according to the present invention, selenious acid, sodium selenate, potassium selenate, magnesium selenate, and calcium selenate are preferable, and selenious acid and sodium selenate are particularly preferable. A content of a selenite compound according to the present invention in the culture medium for producing extracellular vesicles according to the embodiment of the present invention is, for example, 0.001 µg/L to 0.1 mg/L, preferably 0.005 µg/L to 0.05 mg/L, more preferably 0.01 µg/L to 0.01 mg/L, still more preferably 0.05 µg/L to 0.005 mg/L, and particularly preferably 0.1 µg/L to 0.001 mg/L. One or two or more kinds of the selenious acid or a salt thereof according to the present invention may be used.

Examples of the insulin according to the present invention include naturally occurring insulin, recombinant insulin (recombinant form), insulin obtained by a chemical synthesis method, and the like. Examples of the organism from which the naturally occurring insulin is derived include humans, mice, and the like. A content of the insulin according to the present invention in the culture medium for producing extracellular vesicles according to the embodiment of the present invention is, for example, 0.05 mg/L to 500 mg/L, preferably 0.1 mg/L to 250 mg/L, more preferably 0.5 mg/L to 100 mg/L, still more preferably 1 mg/L to 50 mg/L, and particularly preferably 5 mg/L to 25 mg/L.

Examples of the insulin-like growth factors (hereinafter, may be abbreviated as IGFs) according to the present invention include IGF-1, IGF-2, and the like, and IGF-1 is preferable. In addition, examples of the insulin-like growth factors according to the present invention include naturally occurring insulin-like growth factors, recombinant insulin-like growth factors (recombinant form), insulin-like growth factors obtained by a chemical synthesis method, and the like. Examples of the organism from which the naturally occurring insulin is derived include humans, mice, and the like. A content of the insulin-like growth factors according to the present invention in the culture medium for producing extracellular vesicles according to the embodiment of the present invention is, for example, 0.05 µg/L to 50 mg/L, preferably 0.1 µg/L to 10 mg/L, more preferably 0.5 µg/L to 5 mg/L, still more preferably 0.001 mg/L to 1 mg/L, and particularly preferably 0.005 mg/L to 0.5 mg/L.

Examples of the serotonin compound according to the present invention include serotonin, a serotonin complex and a salt thereof, and the serotonin, a serotonin-creatinine complex, and salts thereof are preferable, serotonin, a serotonin-creatinine sulfate complex, serotonin sulfate, and serotonin hydrochloride are more preferable, serotonin and a serotonin-creatinine sulfate complex are still more preferable, and a serotonin-creatinine sulfate complex is particularly preferable. A content of the serotonin compound according to the present invention in the culture medium for producing extracellular vesicles according to the embodiment of the present invention is, for example, 0.005 mg/L to 500 mg/L, preferably 0.01 mg/L to 100 mg/L, more preferably 0.05 mg/L to 50 mg/L, still more preferably 0.1 mg/L to 10 mg/L, and particularly preferably 0.5 mg/L to 5 mg/L.

Examples of the transforming growth factor β include TGFβ1, TGFβ3, and the like, and TGFβ3 is preferable. In addition, examples of the transforming growth factor β according to the present invention include naturally occurring transforming growth factor β, recombinant transforming growth factor β (recombinant form), transforming growth factor β obtained by a chemical synthesis method, and the like. Examples of the organism from which the naturally occurring transforming growth factor β is derived include humans, mice, and the like. A content of the transforming growth factor β according to the present invention in the culture medium for producing extracellular vesicles according to the embodiment of the present invention is, for example, 0.01 µg/L to 0.5 mg/L, preferably 0.05 µg/L to 0.1 mg/L, more preferably 0.1 µg/L to 0.1 mg/L, still more preferably 0.5 µg/L to 0.05 mg/L, and particularly preferably 0.001 mg/L to 0.01 mg/L.

The basal medium for culturing animal cells according to the present invention is not particularly limited as long as it is used for cell culturing animal cells, particularly mesenchymal stem cells and the like. The basal medium for culturing animal cells according to the present invention may contain components necessary for survival of animal cells, and examples thereof include those containing inorganic salts, amino acids, sugars, vitamins, and the like. Examples of the inorganic salts include potassium chloride, sodium chloride, calcium chloride, magnesium chloride, copper sulfate, zinc sulfate, manganese sulfate, magnesium sulfate, iron (III) nitrate, calcium nitrate, iron nitrate, disodium hydrogen phosphate, sodium dihydrogen phosphate, sodium hydrogen carbonate, and the like. Examples of the amino acids include L-alanine, L-arginine, L-asparagine, L-aspartic acid, L-cysteine, L-glutamic acid, glycine, L-histidine, L-isoleucine, L-leucine, L-lysine, L-methionine, L-phenylalanine, L-proline, L-serine, L-threonine, L-tryptophan, L-tyrosine, L-valine, and the like. The basal medium for culturing animal cells according to the present invention may contain 4 mM or less of L-glutamine. Examples of the vitamins include ascorbic acid, biotin, D-pantothenic acid, choline, folic acid, myo-inositol, niacinamide, pyrodoxal, riboflavin, thiamine, cyanocobalamine, DL-α-tocopherol, and the like. Examples of the saccharides include glucose, galactose, fructose, sucrose, and the like. Specific examples of the basal medium for culturing animal cells according to the present invention include basal media for culturing animal cells such as Dulbecco's modified Eagle's medium (DMEM), DMEM/F-12, Ham's F-12, Ham's F-12K, Ham's F-10, Eagle's minimum essential medium (MEM), EMEM (Eagle's minimum essential medium), α-modified Eagle's minimum essential medium (α-MEM), Glasgow minimum essential medium (GMEM), Iscove's modified Dulbecco's medium (IMDM), RPMI-1640 (the Roswell Park Memorial Institute 1640 medium), ATCC-CRCM30, Eagle's basal medium (BME), Fisher's medium, McCoy's 5A medium, Leibovitz's L-15 medium, RITC80-7 medium, MCDB105 medium, MCDB107 medium, MCDB131 medium, MCDB153 medium, MCDB201 medium, NCTC109 medium, NCTC135 medium, Waymouth's MB 752/1 medium, CMRL-1066 medium, Williams' medium E, and ReproFF2 cell culture medium (REPROCELL Inc.), and DMEM, DMEM/F-12, Ham's F-12, Ham's F-12K, Ham's F-10, MEM, EMEM, α-MEM, GMEM, IMDM, RPMI-1640 are preferable, DMEM, DMEM/F-12, Ham's F-12, Ham's F-12K, and Ham's F-10 are more preferable, DMEM and DMEM/F-12 are still more preferable, and DMEM is most preferable. The basal medium for culturing animal cells according to the present invention may be a mixed culture medium obtained by mixing one or two or more of basal media for culturing animal cells, or a mixed culture medium mixed with another culture medium. Modification of a basal medium for culturing animal cells may be applied to the basal medium for culturing animal cells according to the present invention to be suitable for the production of extracellular vesicles. As the animal cells, the mesenchymal stem cells according to the present invention are preferable. In addition, as the origin of the animal cell, a mammal is preferable, and a human is more preferable.

Examples of the culture medium for producing extracellular vesicles according to the embodiment of the present invention include those containing one kind, two kinds, three kinds, four kinds, five kinds, six kinds, and seven kinds of the components according to the present invention, and two or more kinds of the components according to the present invention is preferably contained, and seven kinds of the components are most preferably contained. Examples of the culture medium containing one component according to the present invention include a culture medium containing L-glutamine or a salt thereof of 5 mM or more, a culture medium containing transferrin, a culture medium containing selenious acid or a salt thereof, a culture medium containing insulin, a culture medium containing insulin-like growth factors, a culture medium containing a serotonin compound, and a culture medium containing transforming growth factor β, and a culture medium containing L-glutamine or a salt thereof of 5 mM or more, a culture medium containing insulin-like growth factors, a culture medium containing a serotonin compound are preferable, and a culture medium containing L-glutamine or a salt thereof of 5 mM or more is particularly preferable. As a culture medium containing two components according to the present invention, a culture medium containing transferrin and selenious acid or a salt thereof, and a culture medium containing insulin-like growth factors and transforming growth factor β are preferable. As a culture medium containing three components according to the present invention, a culture medium containing transferrin, selenious acid or a salt thereof, and insulin, and a culture medium containing L-glutamine or a salt thereof of 5 mM or more, insulin-like growth factors and transforming growth factor β are preferable. As a culture medium containing seven components according to the present invention, a culture medium containing L-glutamine or a salt thereof of 5 mM or more, transferrin, selenious acid or a salt thereof, insulin, insulin-like growth factors, a serotonin compound, and transforming growth factor β can be mentioned. As the culture medium for producing extracellular vesicles according to the embodiment of the present invention, a culture medium containing L-glutamine or a salt thereof of 5 mM or more, a culture medium containing insulin-like growth factors, a culture medium containing a serotonin compound; a culture medium containing transferrin and selenious acid or a salt thereof, a culture medium containing insulin-like growth factors and transforming growth factor β; a culture medium containing transferrin, selenious acid or a salt thereof, and insulin, a culture medium containing L-glutamine or a salt thereof of 5 mM or more, insulin-like growth factors, and transforming growth factor β; a culture medium containing L-glutamine or a salt thereof of 5 mM or more, transferrin, selenious acid or a salt thereof, insulin, insulin-like growth factors, a serotonin compound, and a transforming growth factor β are preferable. In addition, as the culture medium for producing extracellular vesicles according to the embodiment of the present invention, the basal medium for culturing animal cells is one culture medium selected from the group consisting of DMEM, DMEM/F-12, Ham's F-12, Ham's F-12K, Ham's F-10, MEM, EMEM, α-MEM, GMEM, IMDM, and RPMI-1640, and is preferably a culture medium containing one component according to the present invention selected from L-glutamine or a salt thereof; insulin-like growth factors; a serotonin compound; transferrin and selenious acid or a salt thereof; insulin-like growth factors and transforming growth factor β; transferrin, selenious acid or a salt thereof, and insulin; L-glutamine or a salt thereof, insulin-like growth factors, and a transforming growth factor β; L-glutamine or a salt thereof, transferrin, selenious acid or a salt thereof, insulin, insulin-like growth factors, a serotonin compound, and transforming growth factor β; or a combination thereof, and in a case of containing L-glutamine or a salt thereof, a concentration in the culture medium for producing extracellular vesicles according to the embodiment of the present invention is 5 mM or more.

The culture medium for producing extracellular vesicles according to the embodiment of the present invention may further contain any components in addition to the components according to the present invention. Examples of any components include reducing agents (for example, 2-mercaptoethanol, catalase, superoxide dismutase, N-acetylcysteine, and the like), organic acids (for example, pyruvic acid, lactic acid, and the like), trace metals (sodium, potassium, magnesium, calcium, iron, cobalt, nickel, copper, zinc, selenium, tin, molybdenum, silicon, and the like), antibiotics (for example, streptomycin, penicillin, gentamycin, canamycin, hyglomycin, and the like), steroids (for example, β-estradiol, progesterone, teststerone, cortisone, cortisol, hydrocortisone, and the like), polyamines (for example, putrescine and the like), buffers (for example, HEPES, sodium hydrogen carbonate, and the like), pH indicators (phenol red), ethanolamine, and the like.

The culture medium for producing extracellular vesicles according to the embodiment of the present invention may contain additives used for culturing cells such as animal cells, particularly mesenchymal stem cells, in addition to those exemplified. These components are preferably contained within a concentration range known per se depending on the purpose for using these components.

### <Additives for Producing Extracellular Vesicles>

The additive for producing extracellular vesicles according to the embodiment of the present invention contains at least one component selected from L-glutamine or a salt thereof, transferrin, selenious acid or a salt thereof, insulin, insulin-like growth factors, a serotonin compound, and transforming growth factor β, and in a case of containing L-glutamine or a salt thereof, the additive contains L-glutamine or a salt thereof with a concentration of 5 mM or more in the culture medium for producing extracellular vesicles.

The additive for producing extracellular vesicles according to the embodiment of the present invention may be a liquid form such as an aqueous solution or a dispersion liquid in which each of the components according to the present invention contained in the additive for producing extracellular vesicles according to the embodiment of the present invention is dissolved or dispersed in a solvent such as water, may be a solid form such as powder or particles, or may have a form different for each component according to the present invention. The additive for producing extracellular vesicles of the present invention may be a substance different for each of the components according to the present invention, or two or more components according to the present invention may be combined and contained, and a substance composed of a combination of the two or more components according to the present invention is preferable. The additive for producing extracellular vesicles according to the present invention is used for producing (preparing) the culture medium for producing extracellular vesicles according to the embodiment of the present invention, in other words, used in a method for producing extracellular vesicles according to the embodiment of the present invention described later, and the additive is mixed with a basal medium for culturing animal cells according to the present invention to be used. The culture medium for producing extracellular vesicles according to the embodiment of the present invention and the basal medium for culturing animal cells according to the present invention are the same as those described above, and specific examples and preferred embodiments are also the same as those described above. The additive for producing extracellular vesicles according to the embodiment of the present invention may contain a component according to the present invention so that a final concentration of the culture medium for producing extracellular vesicles according to the embodiment of the present invention mixed with the basal medium for culturing animal cells according to the present invention is, for example, a content of the component according to the present invention in the culture medium for producing extracellular vesicles according to the embodiment of the present invention. That is, in a case where the basal medium for culturing animal cells according to the present invention contains the component according to the present invention, the additive for producing extracellular vesicles according to the embodiment of the present invention may contain the component of the present invention in an amount obtained by subtracting the content (concentration) of the component according to the present invention contained in the basal medium for culturing animal cells according to the present invention from the content (concentration) of the component according to the present invention in the above-described culture medium for producing extracellular vesicles according to the embodiment of the present invention. For example, in a case where D-MEM (a culture medium containing 4 mM of glutamine) is used as the basal medium for culturing animal cells according to the present invention, an amount calculated by subtracting 4 mM from the content of L-glutamine or a salt thereof according to the present invention in the culture medium for producing extracellular vesicles according to the embodiment of the present invention exemplified above may be used. In addition to the components according to the present invention, the additive for producing extracellular vesicles according to the embodiment of the present invention is a reagent that is usually used in the related art, such as a buffer, a sensitizer, a surfactant, a preservative (for example, sodium azide, salicylic acid, benzoic acid, or the like), a stabilizer (for example, albumin, globulin, water-soluble gelatin, surfactant, sugar, or the like), an activator, an agent for avoiding the influence of coexisting substances, and others used in the related art. In addition, the additive for producing extracellular vesicles according to the embodiment of the present invention may contain a substance that does not inhibit the stability with a coexisting reagent or does not inhibit promoting the production of extracellular vesicles caused by the component according to the present invention. In addition, as for a concentration range and the like of these reagents and the like, the reagents and the like may be appropriately selected and used within a concentration range and the like in which each of the reagents is effective.

### <Culture Medium Kit>

The culture medium kit according to the embodiment of the present invention contains a basal medium for culturing animal cells according to the present invention, and an additive for producing extracellular vesicles containing at least one component selected from L-glutamine or a salt thereof, transferrin, selenious acid or a salt thereof, insulin, insulin-like growth factors, a serotonin compound, and transforming growth factor β, and in a case where the additive for producing extracellular vesicles contains L-glutamine or a salt thereof, the additive for producing extracellular vesicles contains L-glutamine or a salt thereof in an amount at which a concentration of the L-glutamine or a salt thereof in the culture medium for producing extracellular vesicles is 5 mM or more. That is, the culture medium kit according to the embodiment of the present invention includes the basal medium for culturing animal cells according to the present invention and the additive for producing extracellular vesicles according to the embodiment of the present invention.

The culture medium kit according to the embodiment of the present invention is used for producing (preparing) the culture medium for producing extracellular vesicles according to the embodiment of the present invention, in other words, is used for a method for producing extracellular vesicles according to the embodiment of the present invention described later. The kit according to the embodiment of the present invention is used by mixing the additive for producing extracellular vesicles according to the embodiment of the present invention contained in the kit with the basal medium for culturing animal cells according to the present invention. The culture medium for producing extracellular vesicles according to the embodiment of the present invention, the additive for producing extracellular vesicles according to the embodiment of the present invention, the basal medium according to the present invention, and the like, which are included in the culture medium kit according to the embodiment of the present invention, are the same as those described above, and specific examples and preferred embodiments are also the same as those described above.

The culture medium kit according to the embodiment of the present invention may contain one or two or more additives for producing extracellular vesicles according to the embodiment of the present invention in one container, and two or more additives for producing extracellular vesicles according to the embodiment of the present invention are preferably contained in one container.

The kit according to the embodiment of the present invention may further contain, for example, a culture medium for proliferation described later, reagents used in a method for acquiring extracellular vesicles from the sample, an instruction manual, and the like. Examples of the reagents used in the method for acquiring extracellular vesicles from the sample include a kit for acquiring extracellular vesicles, such as MagCapture (trade mark) exosome isolation kit PS (FUJIFILM Wako Pure Chemical Corporation) and reagents constituting the kit, and a kit for concentrating extracellular vesicles, such as ExoQuick Exposome Precision Solution (System Biosciences, LLC.) and reagents constituting the kit, and the like.

### <Method for Producing Extracellular Vesicles>

The method for producing extracellular vesicles according to the embodiment of the present invention is a method for producing extracellular vesicles including producing extracellular vesicles from mesenchymal stem cells by using the culture medium for producing extracellular vesicles according to the embodiment of the present invention.

The culture medium for producing extracellular vesicles, the mesenchymal stem cells, and the extracellular vesicles of the present invention in the method for producing extracellular vesicles according to the embodiment of the present invention are the same as those described above, and specific examples and preferred embodiments are also the same as those described above.

In the method for producing extracellular vesicles according to the embodiment of the present invention includes culturing mesenchymal stem cells by using, for example, the culture medium for producing extracellular vesicles according to the embodiment of the present invention, the method being carried out in a CO₂ environment of, for example, 2% to 7%, for example, at 35°C to 40°C, preferably at 36°C to 38°C, for example, for 24 hours to 240 hours, and preferably 48 hours to 180 hours. The culture may be static culture or shake culture, and static culture is preferable.

Examples of an incubator used in the method for producing extracellular vesicles according to the embodiment of the present invention include a flask, a dish, a chalet, a microwell plate, a microslide, a chamber slide, a tube, a tray, a culture bag, and a culture tank. In addition, the incubator may have cell adhesion or non-cell adhesion.

As necessary, in the method for producing extracellular vesicles according to the embodiment of the present invention, the mesenchymal stem cells according to the present invention is proliferated using a culture medium for proliferation, and the culture medium is then exchanged to the culture medium for producing extracellular vesicles according to the embodiment of the present invention, thereby producing extracellular vesicles from the mesenchymal stem cells according to the present invention with the culture medium for producing extracellular vesicles according to the embodiment of the present invention as described above. The culture medium for proliferation may be any culture medium as long as it has the proliferation potential of mesenchymal stem cells, and examples thereof include basal media, such as α-MEM, DMEM, DMEM/F-12, Ham's F-12, Ham's F-12K, Ham's F-10, MEM, EMEM, GMEM, IMDM, and other media, and culture media for proliferation of mesenchymal stem cells, such as a mesenchymal stem cell proliferation culture medium 2 (Takara Bio Inc.), KBM ADSC-1 (Kohjin Bio Co., Ltd.), KBM ADSC-2 (Kohjin Bio Co., Ltd.), MSC NutriStem (trade mark) XF medium (Biological Industries), TheraPEAK (trade mark) MSCGM (trade mark) Mesenchymal Stem Cell Growth Medium (Lonza Group AG), Serum Free Medium STKR1 for Primary Mesenchymal Stem Cell (Kanto Chemical Co., Inc), Serum Free Medium STKR2 for Mesenchymal Stem Cell (Kanto Chemical Co., Inc), StemXVivo Xeno-Free Human MSC Expansion Media (R&D Systems), StemXVivo Serum-Free Human MSC Expansion Media (R&D Systems), StemXVivo Mesenchymal Stem Cell Expansion Media (R&D Systems), CnT-Prime MSC Medium Xeno-Free (CELLnTEC), CnT-Prime MSC Medium (CELLnTEC), StemProtm MSC SFM (Thermo Fisher Scientific Inc.), PRIME-XV (trade mark) MSC Expansion XSFM (FUJIFILM Irvine Scientific), MesenCult ACF Plus Culture Kit (STEMCELL Technologies), and other media. The culture medium for proliferation may contain 5% to 20% of serum (for example, FBS), and a culture medium for proliferation containing FBS is preferable. The culture medium for proliferation may be a mixed culture medium with one or two or more of basal media or culture media for proliferation of mesenchymal stem cells, or a mixed culture medium with another culture medium. The culture medium for proliferation may be modified to be suitable for proliferation of mesenchymal stem cells, or may be a culture medium in which one or two or more components are added, removed, increased in an amount, or decreased in an amount.

The proliferation of the mesenchymal stem cells according to the present invention is carried out by culturing mesenchymal stem cells with, for example, a culture medium for proliferation in a CO₂ environment of, for example, 2% to 7%, for example, at 35°C to 40°C, preferably at 36°C to 38°C, for example, for 24 hours to 240 hours, and preferably 48 hours to 120 hours. The culture may be static culture or shake culture. In addition, an incubator used for the proliferation of the mesenchymal stem cells according to the present invention is the same as the incubator used for the method for producing extracellular vesicles according to the embodiment of the present invention.

According to the present invention, extracellular vesicles produced by the method for producing extracellular vesicles according to the embodiment of the present invention, and a method for acquiring extracellular vesicles are provided.

The method (isolating and purifying) for acquiring extracellular vesicles produced by the method for producing extracellular vesicles according to the embodiment of the present invention may be any method capable of acquiring extracellular vesicles from a sample, and examples thereof include an affinity method (for example, a PS-affinity method), a fractional centrifugation method (for example, a pellet down method, a sucrose cushioning method, an ultracentrifugal method such as density gradient centrifugation method), an immunoprecipitation method, a chromatography method (for example, an ion exchange chromatography method, a gel permeation chromatography method), a density gradient method (for example, a sucrose density gradient method), an electrophoresis method (for example, an organella electrophoresis method), a magnetic separation method (for example, a magnetically activated cell sorting (MACS) method), an ultrafiltration concentration method (for example, a nanofilm ultrafiltration concentration method), a percol gradient isolation method, a method using a microfluidic device, a PEG precipitation method, and the like, an affinity method with which extracellular membrane vesicles having a high degree of purity can be acquired or a fractional centrifugation method that enables theoretically unbiased recovery is preferable, an affinity method or an ultracentrifugal method is more preferable, and an affinity method is particularly preferable. As the affinity method, a method of acquiring extracellular vesicles by a method using a substance having an affinity for phosphatidylserine (PS-affinity method) is preferable. The affinity method and the fractional centrifugation method may be carried out, for example, according to a method described in JP2016-088689A. One of these isolation methods may be used alone, or two or more thereof may be used in combination. In addition, isolation by one isolation method may be repeated twice or more.

As the substance having an affinity for phosphatidylserine (hereinafter, may be abbreviated as a "PS-affinity substance"), any substance may be employed as long as it can be specifically bonded to phosphatidylserine composing a membrane of an extracellular vesicle. Examples thereof include Annexin V; MFG-E8; Tim proteins such as Tim1 protein (T cell immunoglobulin mucin domain-containing molecule 1, T-cell immunoglobulin-mucin-domain 1), Tim2 protein (T cell immunoglobulin-mucin domain-containing molecule 2, T-cell immunoglobulin-mucin-domain 2), Tim3 protein (T cell immunoglobulin·mucin domain-containing molecule 3, T-cell immunoglobulin-mucin-domain 3), and Tim4 protein (T cell immunoglobulin·mucin domain-containing molecule 4, T-cell immunoglobulin-mucin-domain 4), and from the viewpoint that extracellular vesicles can be efficiently acquired, Tim proteins are preferable, it is more preferable to be selected from Tim4 protein, Tim3 protein, and Tim1 protein, Tim4 protein and Tim1 protein are still more preferable, and Tim4 protein is particularly preferable.

The PS-affinity method including brining a cell culture supernatant liquid containing extracellular vesicles into contact with a PS-affinity substance in the presence of calcium ions, forming a composite body acquired by combining the extracellular vesicles in the cell culture supernatant liquid with the PS-affinity substance, and separating the PS-affinity substance from the composite body to acquire PS-positive extracellular vesicles. The PS-positive extracellular vesicles refer to PS-positive (PS-containing) extracellular vesicles in which phosphatidylserine on a membrane surface of an extracellular vesicle is considered to be exposed.

Specifically, a preferable method as the PS-affinity method includes following steps:
(1) in the presence of calcium ions, bringing a cell culture supernatant liquid containing extracellular vesicles into contact with a PS-affinity substance in the presence of calcium ions, and forming a composite body by combining PS-positive extracellular vesicles in the cell culture supernatant liquid with the PS-affinity substance;
(2) separating the composite body formed by combining the PS-positive extracellular vesicles with the PS-affinitive substance, which is obtained in (1), from the cell culture supernatant liquid containing the extracellular vesicles;
(3) separating the PS-positive extracellular vesicles from the composite body formed by combining the PS-positive extracellular vesicles with the PS-affinitive substance to acquire the PS-positive extracellular vesicles.

### Examples

Hereinafter, the present invention will be specifically described based on Examples and Comparative Examples, but the present invention is not limited to these examples.

### Experimental Example 1. Culture of Mesenchymal Stem Cells

Poietics (trademark) human mesenchymal stem cells (Lonza.), which are bone marrow-derived mesenchymal stem cells, were cultured in "MEMα (L-glutamine, phenol red contained)" (FUJIFILM Wako Pure Chemical Corporation, this culture medium contains 2 mM of L-glutamine) containing 15% FBS (Selborne Biological Services Pty. Co., Ltd.) used as a proliferation culture medium. Thereafter, the cultured bone marrow-derived mesenchymal stem cells were seeded in a 100 mm cell culture dish (Corning Incorporated) with a cell count of 3 × 10⁵, cultured in a cell culture incubator set under conditions of 5% CO₂ and 37°C for 72 hours, and proliferated to a cell count of 3 × 10⁶.

### Examples 1 to 5. Preparation of Culture Medium for Producing Extracellular Vesicles

### (1) Preparation of Culture Medium for Producing Extracellular Vesicles

bFGF was added to "D-MEM (high glucose) (containing L-glutamine and phenol red)" (FUJIFILM Wako Pure Chemical Corporation, this culture medium contains 4 mM of L-glutamine) to be 0.01 µg/mL, thereby preparing a reference culture medium. Next, based on Table 1 described below, 20 mL of a culture medium containing each additive in the reference culture medium was prepared to have a predetermined concentration. The obtained media will be referred to as "Examination culture medium 1" to "Examination culture medium 5", respectively. L-glutamine 584 (µg/mL) of the additive is 4 (mM), and Examination culture medium 1 contains 8 mM in combination with L-glutamine contained in D-MEM.

### (2) Production of Extracellular Vesicles

The bone marrow-derived mesenchymal stem cells proliferated in Experimental Example 1 were exchanged from the proliferation culture medium to each of Examination culture medium 1 to Examination culture medium 5. Thereafter, the cells were cultured for 120 hours in a cell culture incubator set under conditions of 5% CO₂ and 37°C. The obtained culture supernatant was recovered into a 50 mL centrifuge tube and centrifuged at 2,000 × g for 20 minutes, and the supernatant was recovered.

### (3) Acquisition of Extracellular Vesicles

1 mL of each supernatant recovered in (2) was centrifuged at 110,000 × g for 70 minutes with an ultracentrifuge (Beckman: Optima (trade mark) L-100XP). As a result, pellets were obtained, 1 mL of PBS was added to the obtained pellets, and the mixture was centrifuged again at 110,000 × g for 70 minutes. The finally obtained pellets were dissolved with PBS to which an EV-Save (trademark) extracellular vesicle blocking reagent was added (FUJIFILM Wako Pure Chemical Corporation). The obtained solution is referred to as an extracellular vesicle solution.

### Comparative Example 1. Preparation of Culture Medium for Producing Extracellular Vesicles

### (1) Preparation of Culture Medium for Producing Extracellular Vesicles

The reference culture medium prepared in (1) of Examples 1 to 5 is referred to as "Comparative culture medium 1".

Extracellular vesicles were produced and acquired by the same method as in Example 1 except that Comparative culture medium 1 was used instead of Examination culture medium 1, to acquire an extracellular vesicle solution.

**[Table 1]**

| | Culture medium designation | No. in Fig. 1 | Reference culture medium | Additive | Concentration (µg/L) |
|---|---|---|---|---|---|
| Comparative Example 1 | Comparative culture medium 1 | 1 | D-MEM (containing 4 mM of L-glutamine) + bFGF (0.01 µg/mL) | - | - |
| Example 1 | Examination culture medium 1 | 2 | | L-glutamine | 584 |
| Example 2 | Examination culture medium 2 | 3 | | Transferrin | 5 |
| | | | | Sodium selenite | 0.0002 |
| Example 3 | Examination culture medium 3 | 4 | | Serotonin creatinine sulfate monohydrate | 1.01 |
| Example 4 | Examination culture medium 4 | 5 | | Insulin | 12 |
| | | | | Transferrin | 5 |
| | | | | Sodium selenite | 0.0002 |
| Example 5 | Examination culture medium 5 | 6 | | Insulin-like growth factors (IGF) | 0.01 |

### Experimental Example 2. Measurement of The Number of Particles of Extracellular Vesicles by Nanotracking Analysis Method

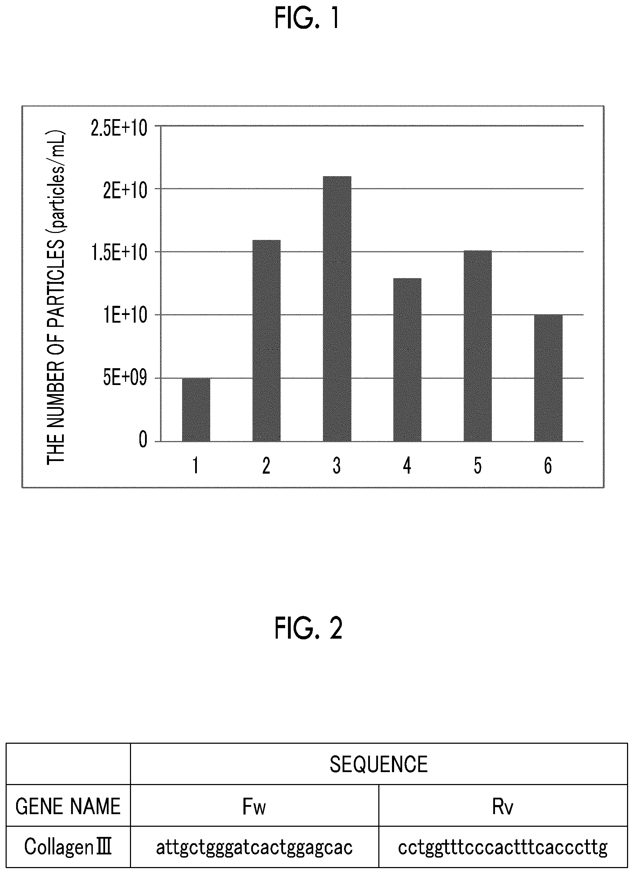

The number of particles of the extracellular vesicle solution obtained in each of Comparative Example 1 and Examples 1 to 5 per unit volume was measured by a nanoparticle tracking analysis method (nano tracking analysis method) using NanoSight (Malvern Panalytical Ltd.) according to a procedure described in the manual of NanoSight, and an average number of particles per unit volume [particles/mL] was calculated. The obtained average number of particles is shown in Fig. 1 and Table 2 below.

**[Table 2]**

| | Culture medium designation | No. in Fig. 1 | Reference culture medium | Additive | Effect of producing extracellular vesicles |
|---|---|---|---|---|---|
| Comparative Example 1 | Comparative culture medium 1 | 1 | D-MEM (containing 4 mM of L-glutamine) + bFGF (0.01 µg/mL) | - | - |
| Example 1 | Examination culture medium 1 | 2 | | L-glutamine | +++ |
| Example 2 | Examination culture medium 2 | 3 | | Transferrin | ++++ |
| | | | | Sodium selenite | |
| Example 3 | Examination culture medium 3 | 4 | | Serotonin creatinine sulfate monohydrate | ++ |
| Example 4 | Examination culture medium 4 | 5 | | Insulin | +++ |
| | | | | Transferrin | |
| | | | | Sodium selenite | |
| Example 5 | Examination culture medium 5 | 6 | | Insulin-like growth factors (IGF) | ++ |

From Fig. 1, as compared with a case of using the culture medium (Comparative culture medium 1) in which bFGF was contained in "D-MEM (high glucose) (containing L-glutamine and phenol red)" (Fujifilm Wako Pure Chemical Corporation, this culture medium contains 4 mM of L-glutamine) so that the culture medium contains 0.01 µg/g of bFGF, in a case of using culture media respectively containing 4 mM of L-glutamine (Example 1), transferrin and sodium selenite (Example 2), serotonin creatinine sulfate monohydrate (Example 3), insulin, transferrin, and sodium selenite (Example 4), and insulin-like growth factors (Example 5), the number of the produced extracellular vesicles was increased significantly. In Table 2, based on the number of the produced extracellular vesicles by using Comparative culture medium 1, a case of 4 times or more was represented by "++++", a case of 3 times or more and less than 4 times was represented by "+++", and a case of 2 times or more and less than 3 times was represented by "++", and the effect of producing extracellular vesicles was evaluated. A larger number of + indicates that the effect of producing extracellular vesicles is higher, and it is preferable as a component of the culture medium for producing extracellular vesicles from the viewpoint of the effect of producing extracellular vesicles. A case of using a culture medium containing transferrin and selenious acid or a salt thereof (Example 2) was represented by "++++", a case of using a culture medium containing L-glutamine or a salt thereof of 5 mM or more (Example 1) was represented by "+++", a case of using a culture medium containing insulin, transferrin, and selenious acid or a salt thereof (Example 4) was represented by "+++", a case of using a culture medium containing a serotonin compound (Example 3) was represented by "++", and a case of using a culture medium containing insulin-like growth factors (Example 3) was represented by "++". From these results, it has been suggested that the culture medium for producing extracellular vesicles containing at least one component selected from L-glutamine or a salt thereof (provided that a concentration thereof in the culture medium is 5 mM or more), transferrin, selenious acid or a salt thereof, insulin, insulin-like growth factors, a serotonin compound, and transforming growth factor β promoted the production of extracellular vesicles.

### Examples 6 to 9. Preparation of Culture Medium for Producing Extracellular Vesicles

### (1) Preparation of Culture Medium for Producing Extracellular Vesicles

bFGF was added to "D-MEM (high glucose) (containing L-glutamine and phenol red)" (FUJIFILM Wako Pure Chemical Corporation, this culture medium contains 4 mM of L-glutamine) to be 0.01 µg/mL, thereby preparing a reference culture medium. Next, based on Table 3 described below, 30 mL of a culture medium containing each additive in the reference culture medium was prepared to have a predetermined concentration. O in Table 3 means that the corresponding component is contained. The obtained media will be referred to as "Examination culture medium 6" to "Examination culture medium 9", respectively.

### (2) Production and Acquisition of Extracellular Vesicles

Extracellular vesicles were produced and acquired by the same method as in Example 1 except that Examination culture medium 6 to Examination culture medium 9 were used instead of Examination culture medium 1, to acquire each of extracellular vesicle solutions.

**[Table 3]**

| | | | Example 6 | Example 7 | Example 8 | Example 9 |
|---|---|---|---|---|---|---|
| Culture medium designation | | | Examination culture medium 6 | Examination culture medium 7 | Examination culture medium 8 | Examination culture medium 9 |
| Reference culture medium | D-MEM (containing 4 mM of L-glutamine) + bFGF (0.01 µg/mL) | | ○ | ○ | ○ | ○ |
| Additive/concentration (µg/mL) | Insulin | 12 | ○ | ○ | ○ | ○ |
| | Sodium selenite | 0.0002 | ○ | ○ | ○ | ○ |
| | Serotonin creatinine sulfate monohydrate | 1.01 | ○ | ○ | ○ | ○ |
| | Insulin-like growth factors (IGF) | 0.01 | ○ | | ○ | ○ |
| | Transferrin | 5 | ○ | ○ | ○ | ○ |
| | Transforming growth factor (TGFβ3) | 0.002 | ○ | ○ | | ○ |
| | L-glutamine | 584 | | | | ○ |

### Experimental example 3. Evaluation of Antifibrinolytic Activity of Extracellular Vesicles

An antifibrinolytic effect of the extracellular vesicles obtained in each of Examples 6 to 9 was evaluated. TIG3 cells (distributed from JCRB), which are human fetal lung-derived fibroblasts, were suspended in DMEM (FUJIFILM Wako Pure Chemical Corporation) containing 10% FBS (Biosera), and were seeded in 24-well plate (Corning Incorporated) with a cell count of 2.5 × 10⁴ per well and a medium volume of 500 µL. 24 hours after cell seeding, each of the extracellular vesicle solution obtained in Example 6 to 10 was added to each well in an amount with a final concentration of 1 × 10⁹ particles/mL and cultured for 16 hours. Thereafter, an amount of TGFβ1 (R&D Systems, Inc.) to obtain a final concentration of 5 ng/mL was added to each well, stimulation was carried out, and furthermore culturing was carried out for 24 hours. Each RNA was extracted by using a PureLink (trademark) RNA Mini kit (Thermo Fisher Scientific Inc.) according to the procedure described in the instruction manual attached to the kit. From 200 ng of the extracted RNA, cDNA was synthesized by using RiverTra Ace (trademark) qPCR RT Master Mix with gDNA Remover (Toyobo Co., Ltd.) according to the procedure described in the instruction manual attached to the kit. The mRNA expression level of the collagen III, which is the fibrosis-related gene, and the internal standard GAPDH was measured by quantitative PCR, by KOD SYBR qPCR Mix (Toyobo Co., Ltd.) using the synthesized cDNA. Primers used for quantitative PCR are illustrated in Fig. 2 (SEQ ID NO. 1 and 2). The measurement results are illustrated in Table 3. As for the expression level of the collagen III gene, the numerical value obtained from qPCR performed with the primer for each gene was normalized to GAPDH, and illustrated as a relative value (ΔΔct value) to the target mRNA expression in the control on the vertical axis.

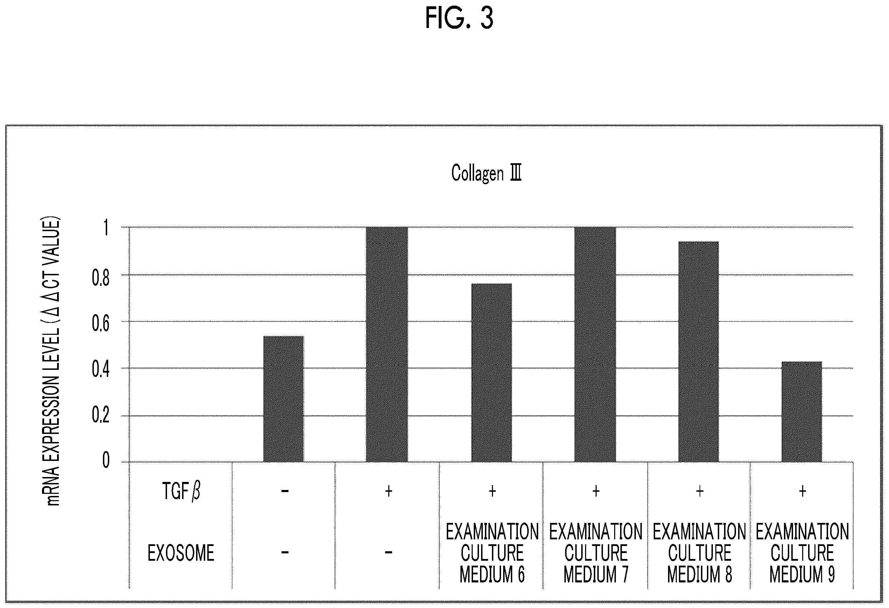

From Fig. 3, the extracellular vesicles produced in the culture medium containing no insulin-like growth factors (IGF) (Examination culture medium 7) and the culture medium containing no transforming growth factor (Examination culture medium 8) showed a lower anti-inflammatory activity than the extracellular vesicles produced by the culture medium containing insulin-like growth factors and transforming growth factor (Examination culture medium 6). Furthermore, the extracellular vesicles produced in the culture medium in which a final concentration of L-glutamine is 8 mM (Examination culture medium 9) showed a higher anti-inflammatory activity than the extracellular vesicles produced by the culture medium containing 4 mM of L-glutamine (only 4 mM contained in the reference culture medium, Examination culture medium 6). From these results, it has been suggested that the culture medium for producing extracellular vesicles containing at least one component selected from L-glutamine or a salt thereof (provided that a concentration thereof in the culture medium is 5 mM or more), insulin-like growth factors and transforming growth factor β promoted the production of extracellular vesicles having the high activity.

### Comparative Example 2 and Examples 10 to 12. Preparation of Culture Medium for Producing Extracellular Vesicles

### (1) Preparation of Culture Medium for Producing Extracellular Vesicles

"D-MEM (high glucose) (L-glutamine-free, phenol red-containing)" (Fujifilm Wako Pure Chemical Corporation) was used as a reference culture medium (Comparative culture medium 2), and based on Table 4 below, 30 mL of a culture medium in which each additive was added to Comparative culture medium 2 to be in a predetermined concentration was prepared. The obtained media will be referred to as "Examination culture medium 10" to "Examination culture medium 12", respectively.

### (2) Production and Acquisition of Extracellular Vesicles

Extracellular vesicles were produced and acquired by the same method as in Example 1 except that Comparative culture medium 2, or Examination culture medium 10 to Examination culture medium 12 were used instead of Examination culture medium 1, to acquire each of extracellular vesicle solutions.

**[Table 4]**

| | Culture medium designation | Reference culture medium | Additive | Additive amount |
|---|---|---|---|---|
| Comparative Example 2 | Comparative culture medium 2 | D-MEM (containing no L-glutamine) | - | - |
| Example 10 | Examination culture medium 10 | | Insulin-like growth factors (IGF) | 10 (µg/L) |
| Example 11 | Examination culture medium 11 | | Transforming growth factor (TGFβ3) | 2 (µg/L) |
| Example 12 | Examination culture medium 12 | | L-glutamine | 8 mM (1.17g/L) |
| Example 5 | Examination culture medium 13 | | EGF | 5 (µg/L) |

### Experimental example 4. Evaluation of Antifibrinolytic Activity of Extracellular Vesicles

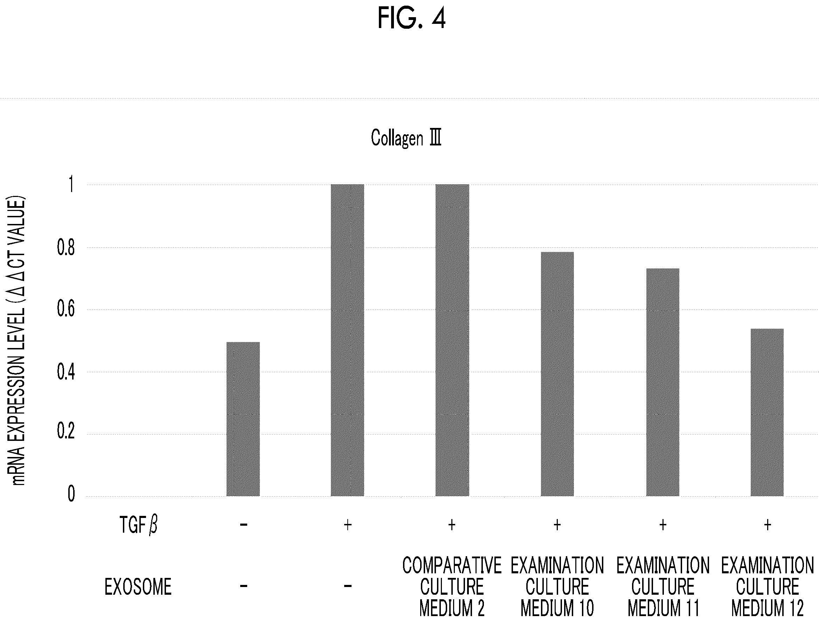

The mRNA expression level of Collagen III, which is a fibrosis-related gene, was measured by quantitative PCR according to the same method as in Experimental Example 3, except that the extracellular vesicle solutions obtained in Comparative Example 2 and Examples 10 to 12 were used, to evaluate the antifibrinolytic effect. The obtained results are illustrated in Fig. 4. From Fig. 4, extracellular vesicles produced in the culture medium containing each of insulin-like growth factors (IGF), transforming growth factor (TGF) β, and L-glutamine were found to have the antifibrinolytic activity, which was not observed in the case of using Comparative culture medium 2. These results have suggested that the culture medium for producing extracellular vesicles containing at least one component selected from IGF, TGFβ, and L-glutamine promoted the production of extracellular vesicles having high activity.

### Experimental example 5. Preparation of Culture Medium for Producing Extracellular Vesicles and Evaluation of Antifibrinolytic Activity

Based on Table 4 described above, 30 mL of a culture medium containing 5 (µg/L) of EGF in Comparative culture medium 2 was prepared, and the antifibrinolytic activity was evaluated by the same method as in Experimental Example 4. The extracellular vesicles produced in the culture medium containing EGF were found to have the antifibrinolytic activity, which was not observed in the case of using Comparative culture medium 2.

The present invention is to provide the culture medium for producing extracellular vesicles, the culture medium kit, the additive, and the method for producing extracellular vesicles. According to the present invention, the present invention is useful, for example, in the field of diagnosis of diseases using extracellular vesicles or research for that purpose because the production of extracellular vesicles is promoted.
[Sequence Table] International Application F21743A-WO CLTLTURE MEDIUM FOR PRODUCING EXTRACELLULAR VESICLES, CULTURE MEDIUM KIT under Patent Cooperation Treaty JP21038095 20211014----00080138152102212777 normal 20211014154034202109300942341330_P1AP101_F2_4.app

## Claims

1. A culture medium for producing extracellular vesicles, comprising:
a basal medium for culturing animal cells; and
at least one component selected from L-glutamine or a salt thereof, transferrin, selenious acid or a salt thereof, insulin, insulin-like growth factors, a serotonin compound, and transforming growth factor β,
wherein a concentration of L-glutamine or a salt thereof is 5 mM or more in the culture medium for producing extracellular vesicles in a case of containing L-glutamine or a salt thereof.

2. The culture medium for producing extracellular vesicles according to claim 1, wherein the culture medium for producing extracellular vesicles contains the L-glutamine or a salt thereof.

3. The culture medium for producing extracellular vesicles according to claim 1, wherein the culture medium for producing extracellular vesicles contains the transferrin.

4. The culture medium for producing extracellular vesicles according to claim 1, wherein the culture medium for producing extracellular vesicles contains the selenious acid or a salt thereof.

5. The culture medium for producing extracellular vesicles according to claim 1, wherein the culture medium for producing extracellular vesicles contains the transferrin and the selenious acid or a salt thereof.

6. The culture medium for producing extracellular vesicles according to claim 1, wherein the culture medium for producing extracellular vesicles contains the insulin.

7. The culture medium for producing extracellular vesicles according to claim 1, wherein the culture medium for producing extracellular vesicles contains the insulin-like growth factors.

8. The culture medium for producing extracellular vesicles according to claim 1, wherein the culture medium for producing extracellular vesicles contains the serotonin compound.

9. The culture medium for producing extracellular vesicles according to claim 1, wherein the culture medium for producing extracellular vesicles contains the transforming growth factor β.

10. The culture medium for producing extracellular vesicles according to claim 1, wherein the culture medium for producing extracellular vesicles does not contain a serum.

11. The culture medium for producing extracellular vesicles according to claim 1, wherein the culture medium for producing extracellular vesicles contains at least two components selected from L-glutamine or a salt thereof, transferrin, selenious acid or a salt thereof, insulin, insulin-like growth factors, a serotonin compound, and transforming growth factor β.

12. The culture medium for producing extracellular vesicles according to claim 1, wherein the culture medium for producing extracellular vesicles contains at least three components selected from L-glutamine or a salt thereof, transferrin, selenious acid or a salt thereof, insulin, insulin-like growth factors, a serotonin compound, and transforming growth factor β.

13. A culture medium kit for producing extracellular vesicles, comprising:
a basal medium for culturing animal cells; and
at least one component selected from L-glutamine or a salt thereof, transferrin, selenious acid or a salt thereof, insulin, insulin-like growth factors, a serotonin compound, and transforming growth factor β,
wherein in a case where the additive for producing extracellular vesicles contains L-glutamine or a salt thereof, the additive for producing extracellular vesicles contains L-glutamine or a salt thereof in an amount at which a concentration of the L-glutamine or a salt thereof in the culture medium for producing extracellular vesicles is 5 mM or more.

14. An additive for producing extracellular vesicles used to produce a culture medium,
wherein the culture medium is the culture medium according to any one of claims 1 to 12,
the additive for producing extracellular vesicles contains at least one component selected from L-glutamine or a salt thereof, transferrin, selenious acid or a salt thereof, insulin, insulin-like growth factors, a serotonin compound, and transforming growth factor β, and
in a case where the additive for producing extracellular vesicles contains L-glutamine or a salt thereof, the additive for producing extracellular vesicles contains L-glutamine or a salt thereof in an amount at which a concentration of the L-glutamine or a salt thereof in the culture medium for producing extracellular vesicles is 5 mM or more.

15. A method for producing extracellular vesicles, the method comprising:
producing extracellular vesicles from mesenchymal stem cells by using the culture medium according to any one of claims 1 to 12.
